# EUROPÄISCHE PATENTSCHRIFT

(11) **EP 1 340 445 B1**
(45) Veröffentlichungstag und Bekanntmachung des Hinweises auf die Patenterteilung: **30.07.2008**
(21) Anmeldenummer: 03450040.5
(22) Anmeldetag: 14.02.2003
(51) Int. Cl.: A47L 9/18

(54) **Staubsauger**
Vacuum cleaner
Aspirateur

(30) Priorität: 27.02.2002 AT 3072002
(43) Veröffentlichungstag der Anmeldung: 03.09.2003
(73) Patentinhaber: Luttermann, Joachim, 8082 Kirchbach (AT)
(72) Erfinder: Luttermann, Joachim, 8082 Kirchbach (AT)
(74) Vertreter: Gibler, Ferdinand

(56) Entgegenhaltungen:
- US-A- 4 048 032
- US-A1- 2001 034 922
- PATENT ABSTRACTS OF JAPAN Bd. 1998, Nr. 08, 30. Juni 1998 (1998-06-30) & JP 10 057282 A (SHARP CORP), 3. März 1998 (1998-03-03)
- PATENT ABSTRACTS OF JAPAN Bd. 017, Nr. 496 (C-1108), 8. September 1993 (1993-09-08) & JP 05 130955 A (AKAI ELECTRIC CO LTD), 28. Mai 1993 (1993-05-28)

## Beschreibung

Die Erfindung betrifft einen Staubsauger mit einem Saug-Aggregat und einem Filterbehälter, durch den die über das Saug-Aggregat eingesaugte Luft hindurchgeleitet und von Staub befreit wird, welcher Filterbehälter durch einen mit einer Filterflüssigkeit, z.B. Wasser, zumindest teilweise gefüllt ausgebildet ist.

Bekannte Staubsauger mit Flüssigkeitsfilter ermöglichen zwar eine Herabsetzung der durch den vom Staubsauger ausgestoßenen Luftstrom hervorgerufenen Staubbelastung, in der Filterflüssigkeit kommt es aber bereits nach kurzer Betriebszeit zu einer Vermehrung der dort zurückgehaltenen Bakterien und Krankheitserregern, die dann zusammen mit vom Luftstrom mitgerissenen Wassertröpfchen an die Umwelt gelangen können und somit eine Gesundheitsgefahr darstellen. Ein Austausch der Filterflüssigkeit kann aber aus Gründen der Bedienerfreundlichkeit nicht allzu oft erfolgen.

Aus diesem Grund mußte bei vielen der bereits entwickelten Staubsauger mit Flüssigkeitsfiltern von einer Produktion abgesehen werden. Derartige Staubsauger wurden bereits in JP 5 130 955-A offenbart.

Aufgabe der Erfindung ist es daher, einen Staubsauger der eingangs genannten Art anzugeben, bei welchem die Wirkungsdauer der Filterflüssigkeit stark erhöht und der Ausstoß von gesundheitsgefährlichen Keimen stark herabgesetzt wird.

Erfindungsgemäß wird dies dadurch erreicht, daß im Filterbehälter zumindest zwei mit der Filterflüssigkeit in Kontakt stehende, vorzugsweise in diese eintauchende, Elektroden aus Silber oder einer Silber-Legierung angeordnet sind, und daß eine Spannungsquelle vorgesehen ist, welche mit den Elektroden verbindbar ist.

Die durch Anlegen einer Spannung an die Elektroden in der Filterflüssigkeit gebildeten Silber-Ionen verhindern aufgrund ihrer desinfizierenden Wirkung das Entstehen von Keimund Bakterienpopulationen. Auf diese Weise behält die Filterflüssigkeit ihre staubabscheidende Wirkung im Vergleich zu bekannten Staubsaugern dieser Art über einen sehr langen Zeitraum bei und eine Gefährdung der Umwelt durch Filterflüssigkeitströpfchen, die vom ausgestoßenen Luftstrom mitgerissen werden, kann dadurch verhindert werden.

Eine starke Erhöhung der keimtötenden Wirkung kann erzielt werden, indem gemäß einer weiteren Ausführungsform der Erfindung die Spannungsquelle, vorzugsweise niederfrequente, Gleichspannungsimpulse erzeugt. Der dadurch innerhalb der Filterflüssigkeit bewirkte pulsierende Strom erhöht die Anzahl der durch Silber-Ionen vernichteten pathogenen Keime.

Eine Ausführungsform der Erfindung kann darin bestehen, daß die Frequenz der Gleichspannungsimpulse im Bereich von 2 bis 6 Hz, vorzugsweise 4 Hz, beträgt. Der genannte Bereich, insbesondere der Wert von 4 Hz hat weitgehend unabhängig von der Höhe der angelegten Spannung eine gegenüber anderen Frequenzen besonders starke Herabsetzung der in der Filterflüssigkeit enthaltenen Keime und Bakterien zur Folge.

Um den üblichen sicherheitstechnischen Standards gerecht zu werden, kann in Weiterbildung der Erfindung die Spannungsquelle eine Batterie oder einen Akkumulator umfassen, der mit einem Gleichspannungswandler verbunden ist.

Auf diese Weise ist eine völlige galvanische Trennung zwischen der Netzspannung und den mit einer Spannung beaufschlagten Silberelektroden möglich.

Eine weitere Variante der Erfindung kann darin bestehen, daß die Batterie eine 9V-Batterie ist, und daß der Gleichspannungswandler eine Ausgangsspannung von 30V aufweist, die mit einer Impulsfrequenz von 4Hz getaktet ist.

Dadurch kann eine überall käuflich erhältliche Batterie zum Betreiben der Wasserentkeimung verwendet werden, wobei alle relevanten Sicherheitsstandards eingehalten werden können.

Alternativ dazu kann eine weitere Ausführungsform der Erfindung darin bestehen, daß die Spannungsquelle einen Trenntransformator, einen Gleichrichter und einen Impulsformer umfaßt. Der Trenntransformator erfüllt ebenfalls die gängigen sicherheitstechnischen Auflagen.

Eine weitere Verstärkung der keimtötenden Wirkung läßt sich gemäß einer weiteren Ausbildung der Erfindung dadurch erreichen, daß der Filterbehälter eine Öffnung enthält, über welche dem Filterwasser eine Natriumchlorid-Lösung und/oder eine Silberkolloidlösung zusetzbar ist.

Die durch den zwischen den Elektroden fließenden Strom gebildeten Chlor-Ionen stellen eine Hilfe zur Beseitigung der zu Beginn des Stromflusses in der Filterflüssigkeit vorhandenen Keime dar, da der Entkeimungsprozeß auf Basis der Silber-Ionen eine längere Anlaufzeit benötigt. Die Zugabe einer Silberkolloidlösung erhöht hingegen die Anzahl der für die Entkeimung wirksamen Silber-Ionen in der Filterflüssigkeit.

Nachfolgend wird die Erfindung anhand des in der Zeichnung dargestellten Ausführungsbeispiels eingehend erläutert. Es zeigt dabei

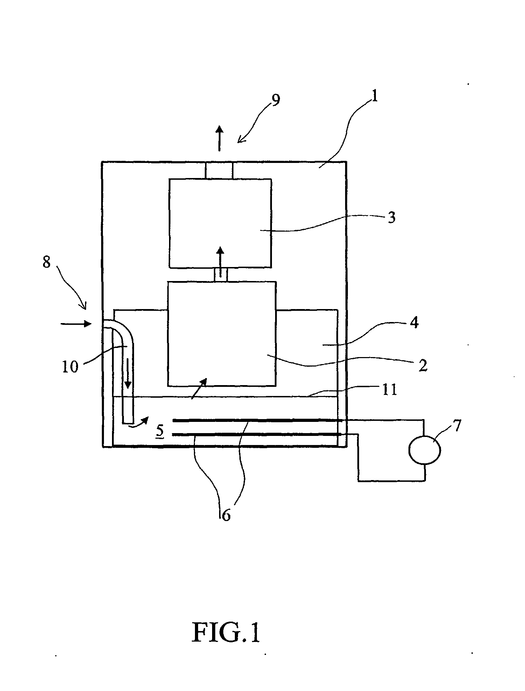

Fig. 1 den schematischen Aufbau einer Ausführungsform des erfindungsgemäßen Staubsaugers.

Fig.1 zeigt einen Staubsauger 1, der über einen Einlaß 8, der z.B. über einen nicht dargestellten Schlauch mit einer Staubsauger-Bürste verbunden ist, kleine Teilchen, z.B. Schmutzteilchen ansaugt, die zusammen mit dem angesaugten Luftstrom durch einen Filterbehälter 4 hindurchgeleitet werden, der mit einer Filterflüssigkeit, z.B. Wasser 5, zumindest teilweise gefüllt ist. Beim Durchströmen der Filterflüssigkeit 5 wird der im angesaugten Luftstrom beinhaltete Staub und Schmutz im Wasser gebunden und dort zurückgehalten. Nach einiger Betriebszeit des Staubsaugers 1 muß die Filterflüssigkeit 5 ausgetauscht werden.

Der über den Einlaß 8 eingesaugte und in einem Rohrstutzen 10 in die Filterflüssigkeit 5 eingeleitete Luftstrom wird durch ein Saugaggregat 3 erzeugt, das im oberen Teil des Staubsaugers 1 angeordnet ist. Zwischen dem Saugaggregat 3 und dem Flüssigkeitsspiegel 11 der Filterflüssigkeit 5 innerhalb des Filterbehälters 4 ist ein zusätzlicher, optionaler Teilchenfilter 2 angeordnet, der beim Austreten des Luftstromes aus der Filterflüssigkeit 5 emporgeschleuderte Teilchen zurückhält. Der durch das Saugaggregat 3 ausgebildete Unterdruck ruft den durch die Filterflüssigkeit 5 hindurchströmenden Luftstrom hervor, der über den Einlaß 8 und den Rohrstutzen 10 in den Staubsauger 1 eintritt und nach Durchströmen der Filterflüssigkeit 5 durch den Teilchenfilter 4 in das Saugaggregat 3 gesaugt wird.

Am Ausgang des Saugaggregates 3 wird der in der Filterflüssigkeit 5 vom Staub befreite, angesaugte Luftstrom über den Auslaß 9 wieder ausgestoßen, wo er schadstoffvermindert an die Umgebung abgegeben wird. Aufgrund der aus dem Luftstrom in der Filterflüssigkeit 5 ausfallenden Teilchen, die verschiedenste Schadstoffe, wie Schwermetalle usw., aber auch Krankheitserreger und Keime enthalten, beginnt in der Filterflüssigkeit 5 innerhalb kürzester Zeit eine starke Vermehrung dieser Erreger und Keime, die durch eine deutliche Dunkelfärbung der Filterflüssigkeit sichtbar wird. Schließlich führt dieser Prozeß dazu, daß die Filterflüssigkeit 5 ihre reinigende Wirkung fast völlig verliert und im Gegenteil der am Auslaß 9 ausgestoßene Luftstrom Wassertröpfchen enthält, die zusammen mit gefährlichen Keimen und Krankheitserregern an die Umgebung abgeben werden und daher die Funktionsfähigkeit des Staubsaugers schwer beeinträchtigt bzw. diesen zu einem Gesundheitsrisiko werden läßt.

Erfindungsgemäß ist vorgesehen, daß im Filterbehälter 1 zumindest zwei mit der Filterflüssigkeit 5 in Kontakt stehende, vorzugsweise in diese eintauchende, Elektroden 6 aus Silber oder einer Silber-Legierung angeordnet sind. Die Elektroden 6 sind über einen nicht dargestellten Schalter mit einer Spannungsquelle 7 verbunden.

Bei Anlegen einer Spannung an die Elektroden 6 werden mit Hilfe eines dabei fließenden schwachen Stromes Silber-Ionen erzeugt, die aufgrund der bekannten keimtötenden Wirkung des Silbers eine Desinfektion und Entkeimung der Filterflüssigkeit 5 bewirken.

Vorzugsweise erzeugt die Spannungsquelle niederfrequente, Gleichspannungsimpulse, die an die Elektroden 6 angelegt werden und einen mit niedriger Frequenz pulsierenden Strom innerhalb der Filterflüssigkeit 5 zur Folge haben.

Die Frequenz der Gleichspannungsimpulse liegt dabei bevorzugt im Bereich von 2 bis 6 Hz, wobei 4 Hz, bezogen auf ein Gleichspannungs-Rechtecksignal bei durchgeführten Versuchsreihen die besten Ergebnisse hinsichtlich des Grads an Entkeimung gebracht hat.

Um auch übliche sicherheitstechnische Standards erfüllen zu können, dürfen keine für den Anwender gefährlichen Spannungen in der Filterflüssigkeit 5 auftreten.

Es kann daher vorgesehen sein, daß die Spannungsquelle eine Batterie oder einen Akkumulator umfaßt, der mit einem Gleichspannungswandler verbunden ist. So kann die Batterie z.B. eine 9V-Batterie sein, mit deren Hilfe der Gleichspannungswandler eine Ausgangsspannung von 30V erzeugt, die mit einer Impulsfrequenz von 4Hz getaktet ist. So kann etwa ein seitlich am erfindungsgemäßen Staubsauger angebrachtes Gehäuse eine handelsübliche 9V-Batterie enthalten, die auf einfache Weise durch den Anwender ausgetauscht werden kann.

Eine andere Variante der Spannungsversorgung kann darin bestehen, daß die Spannungsquelle einen Trenntransformator, einen Gleichrichter und einen Impulsformer umfaßt, wobei der Trenntransformator an seiner Primärseite mit der Netzspannung und an seiner Sekundärseite mit dem Gleichrichter verbunden ist. Die Ausgangsspannung des Gleichrichters wird dann durch den Impulsformer in eine pulsierende Gleichspannung umgewandelt, die an die Elektroden 6 angelegt wird.

Weiters hat sich herausgestellt, daß die Beigabe von Natriumchlorid zur Filterflüssigkeit, insbesondere Wasser, eine die Entkeimung der Flüssigkeit verbessernde Wirkung hat, weil die durch Elektrolyse entstehenden Chlor-Ionen ebenfalls eine reinigende Wirkung entfalten. Zusätzlich oder alternativ kann der Filterflüssigkeit 5 Silber-Kolloid zugesetzt werden, um die Anzahl der Silber-Ionen in der Filterflüssigkeit 5 zu erhöhen.

Es kann daher im Filterbehälter 4 eine in Fig.1 nicht dargestellte Öffnung ausgebildet sein, über welche der Filterflüssigkeit 5 eine Natriumchlorid-Lösung und/oder eine Silber-Kolloidlösung zusetzbar ist. Alternativ kann die Zugabe der Natriumchlorid-Lösung bzw. des Silber-Kolloids durch Abheben des Saugaggregates 3 vom Staubsauger 1 und direktes Hineinschütten in die Filterflüssigkeit 5 erfolgen. Wird die letztgenannte Lösung bzw. Mischflüssigkeit aus Natriumchlorid-Lösung und/oder Silber-Kolloidlösung in die Filterflüssigkeit 5, insbesondere in das Wasser, gegeben, so kann eine rasch einsetzende Entkeimungswirkung hervorgerufen werden, um die Anfangsphase, in der die durch die Silber-Ionen hervorgerufene Reinigungswirkung erst relativ langsam in Gang gesetzt wird, zu überbrücken.

## Patentansprüche

1. Staubsauger mit einem Saug-Aggregat und einem Staubfilter, durch den die über das Saug-Aggregat eingesaugte Luft hindurchgeleitet und von Staub befreit wird, welcher Staubfilter durch einen mit einer Filterflüssigkeit, z.B. Wasser, zumindest teilweise gefiillten Filterbehälter gebildet ist, **dadurch gekennzeichnet, daß** im Filterbehälter (4) zumindest zwei mit der Filterflüssigkeit (5) in Kontakt stehende, vorzugsweise in diese eintauchende, Elektroden (6) aus Silber oder einer Silber-Legierung angeordnet sind, und daß eine Spannungsquelle (7) vorgesehen ist, welche mit den Elektroden (6) verbindbar ist.

2. Staubsauger nach Anspruch 1, **dadurch gekennzeichnet, daß** die Spannungsquelle (7), vorzugsweise niederfrequente, Gleichspannungsimpulse erzeugt.

3. Staubsauger nach Anspruch 1 oder 2, **dadurch gekennzeichnet, daß** die Frequenz der Gleichspannungsimpulse im Bereich von 2 bis 6 Hz, vorzugsweise 4 Hz, beträgt.

4. Staubsauger nach Anspruch 1, 2 oder 3, **dadurch gekennzeichnet, daß** die Spannungsquelle eine Batterie oder einen Akkumulator umfaßt, der mit einem Gleichspannungswandler verbunden ist.

5. Staubsauger nach Anspruch 4, **dadurch gekennzeichnet, daß** die Batterie eine 9V-Batterie ist, und daß der Gleichspannungswandler eine Ausgangsspannung von 30V aufweist, die mit einer Impulsfrequenz von 4Hz getaktet ist.

6. Staubsauger nach Anspruch 1, 2 oder 3, **dadurch gekennzeichnet, daß** die Spannungsquelle einen Trenntransformator, einen Gleichrichter und einen Impulsformer umfaßt.

7. Staubsauger nach einem der vorhergehenden Ansprüche 1 bis 6, **dadurch gekennzeichnet, daß** der Filterbehälter eine Öffnung enthält, über welche dem Filterwasser eine Natriumchlorid-Lösung und/oder eine Silber-Kolloid-Lösung zusetzbar ist.

## Claims

1. A vacuum cleaner, comprising a suction apparatus and a dust filter, through which the air drawn in by the suction apparatus is guided and is freed from dust, which dust filter is formed by a filter container filled at least partly with a filter liquid such as water, **characterized in that** at least two electrodes (6) which are made of silver or a silver alloy and are in contact with the filter liquid (5), preferably immersing in the same, are arranged in the filter container (4), and that a voltage source (7) is provided which can be connected with the electrodes (6).

2. A vacuum cleaner according to claim 1, **characterized in that** the voltage source (7) generates preferably low-frequency direct voltage pulses.

3. A vacuum cleaner according to claim 1 or 2, **characterized in that** the frequency of the direct voltage pulses is in the range of 2 to 6 Hz, preferably 4 Hz.

4. A vacuum cleaner according to claim 1, 2 or 3, **characterized in that** the voltage source comprises a battery or a storage battery which is connected with a DC converter.

5. A vacuum cleaner according to claim 4, **characterized in that** the battery is a 9V battery and that the DC converter has an output voltage of 30V which is clocked with a pulse frequency of 4Hz.

6. A vacuum cleaner according to claim 1, 2 or 3, **characterized in that** the voltage source comprises an isolating transformer, a rectifier and a pulse shaper.

7. A vacuum cleaner according to one of the preceding claims 1 to 6, **characterized in that** the filter container comprises an opening through which a sodium chloride solution and/or a silver colloid solution can be added to the filter water.

## Revendications

1. Aspirateur avec un groupe d'aspiration et un filtre à poussière à travers lequel l'air aspiré par le groupe d'aspiration est guidé et débarrassé de la poussière, lequel filtre à poussière est formé par un récipient de filtre rempli au moins partiellement avec un liquide filtrant, par exemple de l'eau, **caractérisé en ce que** le récipient de filtre (4) contient au moins deux électrodes (6) en argent ou en alliage d'argent qui sont en contact avec le liquide filtrant (5), de préférence plongées dans celui-ci, et **en ce qu'**il est prévu une source de tension (7) qui peut être reliée aux électrodes (6).

2. Aspirateur selon la revendication 1, **caractérisé en ce que** la source de tension (7) produit de préférence des impulsions de courant continu à basse fréquence.

3. Aspirateur selon la revendication 1 ou 2, **caractérisé en ce que** la fréquence des impulsions de courant continu est de l'ordre de 2 à 6 Hz, de préférence de 4 Hz.

4. Aspirateur selon la revendication 1, 2 ou 3, **caractérisé en ce que** la source de tension comprend une batterie ou un accumulateur qui est relié à un transformateur de courant continu.

5. Aspirateur selon la revendication 4, **caractérisé en ce que** la batterie est une batterie de 9 V et **en ce que** le transformateur de courant continu présente une tension de sortie de 30 V à une fréquence d'impulsions de 4 Hz.

6. Aspirateur selon la revendication 1, 2 ou 3, **caractérisé en ce que** la source de tension comprend un transformateur sectionneur, un redresseur et un circuit de mise en forme d'impulsions.

7. Aspirateur selon l'une des revendications 1 à 6, **caractérisé en ce que** le récipient de filtre présente une ouverture par laquelle l'eau du filtre peut être additionnée d'une solution de chlorure de sodium et/ou d'une solution d'argent colloïdal.
